# EUROPÄISCHE PATENTSCHRIFT

(11) **EP 1 607 066 B1**
(45) Veröffentlichungstag und Bekanntmachung des Hinweises auf die Patenterteilung: **16.11.2011**
(21) Anmeldenummer: 05010333.2
(22) Anmeldetag: 12.05.2005
(51) Int. Cl.: A61F 2/36, A61N 1/32, A61F 2/28, A61F 2/30

(54) **Hüftkopfkappenimplantat mit Vorrichtung zur elektrischen Gewebestimulation**
Femoral head cap implant with device for electric stimulation of tissue
Calotte de tête fémorale implantable avec dispositif pour la stimulation électrique de tissu

(30) Priorität: 14.05.2004 DE 102004024473
(43) Veröffentlichungstag der Anmeldung: 21.12.2005
(73) Patentinhaber: Neue Magnetodyn GmbH, 80333 München (DE)
(72) Erfinder: Kraus, Werner, 80539 München (DE); Kraus-von Wesendonk, Christian, 81675 München (DE); Stephan, Heribert, 80689 München (DE)
(74) Vertreter: Schumacher & Willsau

(56) Entgegenhaltungen:
- EP-A- 0 781 532
- DE-A1- 2 611 744
- US-A- 5 376 122
- US-B1- 6 503 281

## Beschreibung

Die vorliegende Erfindung betrifft ein Hüftkopfkappenimplantat mit einer Vorrichtung zur elektrischen Förderung des Wachstums von Knochengewebe und seiner Erhaltung im vitalen Zustand.

Es ist bekannt, dass das Gewebewachstum insbesondere das Knochenwachstum durch einen niederfrequenten elektrischen Wechselstrom mit einer Frequenz im Bereich von ca 8 bis 20 Hz, gefördert werden kann (Verfahren nach Kraus und Lechner). Der Wechselstrom wird durch mindestens zwei Gewebeelektroden appliziert, die mit den Anschlüssen einer implantierten Spule ("Aufnehmerspule") gekoppelt sind. In der Aufnehmerspule wird der Wechselstrom, analog zu einem Transformator, durch eine äußere Spule induziert, die wiederum durch einen geeigneten Generator mit niederfrequentem Wechselstrom gespeist wird (DE-A-31 32 488). Dieses Verfahren findet auch bei der vorliegenden Erfindung Anwendung.

Es sind auch schon Hüftgelenk-Schaftprothesen bekannt, in die zur Stimulierung des Knochenwachstums und Verhinderung einer Lockerung eine Aufnehmerspule und Gewebeelektroden integriert sind (EP-A-0 781 432).

In einem Prospekt der Fa. midland medical technologies ist ein Hüftkopf-Kappensystem Metall/Metall nach Derek McMinn FRCS beschrieben, bei dem nicht mehr der ganze Schenkelhals durch eine Prothese ersetzt wird, sondern eine Metallkappe auf den für sie passend gefrästen Knochen des Hüftgelenkkopfes aufgesetzt wird. Die Metallkappe wird durch einen an ihrer Innenseite angebrachten, im Schenkelhals implantierten Dom fixiert. Solche Kappenprothesen sind Schaftprothesen darin überlegen, dass sie die Geometrie des Oberschenkelknochens kaum verändern. Wie Versuche in den 80er Jahren gezeigt haben, nekrotisiert jedoch der Hüftkopfknochen unter der Metallkappe.häufig innerhalb von nur drei bis vier Jahren und die Metallkappe verliert dadurch ihren Halt. Sie muss dann gegen eine Hüftgelenk-Schaftprothese mit Verankerung des Schaftes im Oberschenkelknochen ausgetauscht werden.

Die in Verbindung mit Hüftgelenkprothesen bekannten Stimulierungsvorrichtungen der eingangs genannten Art lassen sich wegen der unterschiedlichen Konfiguration nicht auf ein Hüftkopf-Kappensystem übertragen.

Der Erfindung liegt die Aufgabe zugrunde, bei einem Hüftkopf-Kappensystem die Nekrotisierung des mit der Metallkappe abgedeckten Knochens zu verhindern und das Knochengewebe vital zu erhalten.

Die obige Aufgabe wird gemäß der vorliegenden Erfindung durch die in den Ansprüchen unter Schutz gestellten und im Folgenden anhand von Ausführungsbeispielen beschriebenen Maßnahmen gelöst.

Durch die erfindungsgemäße Stimulationsvorrichtung wird der Vorteil erzielt, dass bei einem Hüftkopf-Kappensystem eine Stromverteilung erreicht wird, die die Nekrotisierung des abgedeckten Knochens verhindert und das Knochengewebe vital erhält. Das Problem der Langzeithaltbarkeit von Hüftkopf-Kappenprothesen wird dadurch gelöst.

Die Erfindung wird im Folgenden anhand von Ausführungsbeispielen unter Bezugnahme auf die Zeichnungen näher erläutert. Es zeigen:
Fig. 1 eine Schnittansicht eines Hüftkopfkappenimplantats, das mit einer elektrischen Stimulierungsvorrichtung gemäß der Erfindung versehen ist;
Fig. 2 eine Fig. 1 entsprechende Darstellung einer zweiten Ausführungsform der Erfindung,
Fig. 3 eine Fig. 1 entsprechende Darstellung einer dritten Ausführungsform der Erfindung und
Fig. 4 eine in die Zeichenebene projizierte Darstellung der Innenseite eines Hüftkopfkappenteiles gemäß einer Weiterbildung der Erfindung.

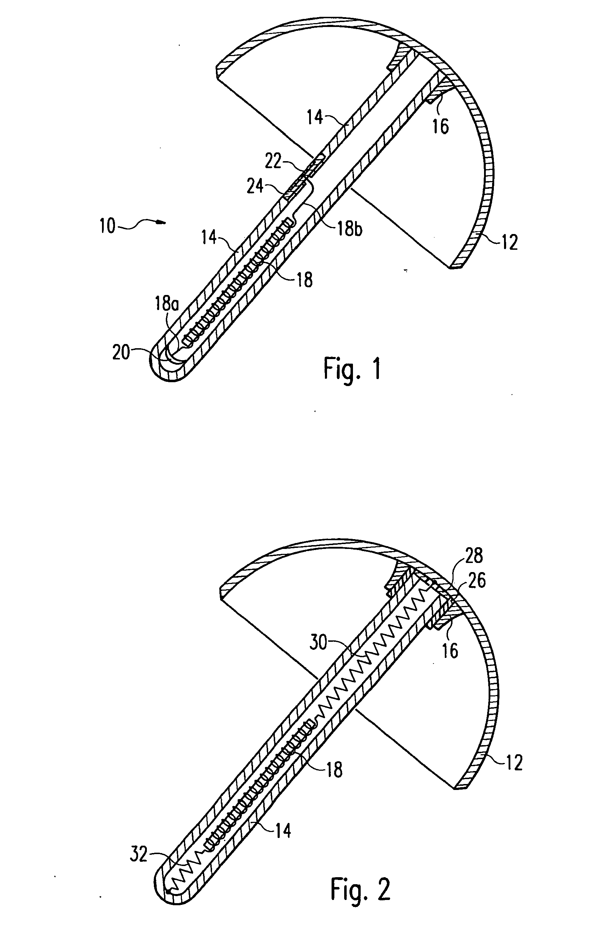

Das in Fig. 1 dargestellte Hüftkopfkappenimplantat 10 enthält ein kalottenförmiges Kappenteil 12 und einen stabförmigen Schaft oder Dom 14. Der Dorn 14 ist mit dem Kappenteil 12 durch eine rohrförmige Halterung 16 verbunden, die an der Innenseite des Kappenteils 12 befestigt ist und das eine Ende des Doms 14 aufnimmt. Der Dom 14 wird in eine Bohrung im Schenkelhalsknochen so eingesetzt, dass das Kappenteil 12 die Oberfläche des entsprechend bearbeiteten Hüftgelenkkopfes abdeckt. Das Kappenteil 12 und der Dom 14 bestehen aus gewebeverträglichem Metall, insbesondere einer Kobalt-Chrom-Molybdän- oder Titan-Aluminium-Vanadium-Legierung. Insoweit ist die Hüftkopfkappenprothese aus dem eingangs erwähnten Prospekt bekannt.

Bei dem in Fig. 1 dargestellten Ausführungsbeispiel der Erfindung ist der Dom 14 ein am freien Ende geschlossenes Rohr, in dem eine Aufnehmerspule 18 untergebracht ist. Die Aufnehmerspule 18 hat einen stabförmigen Magnetkern und eine diesen umgebende Wicklung, deren einer Anschluß 18a über eine Feder 20 elektrisch mit der Innenwand des Domes gekoppelt ist. Dorn 14 und Kappenteil 12 bilden also die eine Gewebeelektrode. Die Außenseite des Doms 14 hat nahe dem vom Kappenteil 12 umschlossenen Raum oder innerhalb von diesem eine Vertiefung. In dieser befindet sich eine Außenelektrode 22, die durch eine dünne Isolierschicht 24 gegen den Dom 14 isoliert ist. Am Ort der Vertiefung hat die Wand des Doms 14 ein Loch. Der andere Anschluß 18b der Aufnehmerspule 18 ist isoliert durch dieses Loch hindurchgeführt und mit der Außenelektrode 22 verbunden. Die Oberfläche der Außenelektrode 22 und des sie umgebenden Randes der Isolierschicht 24 fluchten mit den an die Vertiefung angrenzenden Teile der Oberfläche des Doms 14, so dass die Außenfläche des Doms 14 glatt ist. Die Vertiefung mit der Außenelektrode 22 kann sich über einen Teil des Umfanges des Doms 14 erstrecken oder diesen ringförmig umfassen. Die Elektrode 22 kann aus einer aufgedampften Schicht aus gewebeverträglichem Metall, wie Titan, bestehen. Die Isolierschicht kann aus aufgedampftem Siliciumdioxid oder einem anderen gewebeverträglichen Isoliermaterial bestehen.

Zur Stimulation des Wachstums und zur Vitalerhaltung des Knochens, der durch das Kappenteil abgedeckt ist und in den der Dom eingesetzt ist, wird mittels einer mit einem Wechselstromgenerator gekoppelten äußeren Spule (nicht dargestellt) in der Aufnehmerspule 18 ein niederfrequenter Wechselstrom induziert (Verfahren nach Kraus und Lechner, s: o.).

Bei dem Ausfiihrungsbeispiel gemäß Fig. 2 bilden das Kappenteil 12 die eine und der Dorn 14 die zweite Gewebeelektrode. Der Dorn ist durch eine isolierende Muffe 26, die z. B. aus PTFE bestehen kann, gegen die rohrförmige Halterung 16 isoliert. Eine isolierende Ringscheibe 28 isoliert die Stirnseite des Doms 14 gegen die Innenseite des Kappenteils 12. Die Anschlüsse der Aufnehmerspule 18 sind jeweils über eine Druckfeder 30, 32 mit dem Kappenteil 12 bzw. dem Dom 14 gekoppelt.

Das Innere des Doms 14 der Implantate gem. Fig. 1 und 2 ist gewöhnlich mit isolierender Vergußmasse, insbesondere gewebeverträglichem Epoxidharz, ausgegossen (in Fig. 1 und 2 nicht dargestellt).

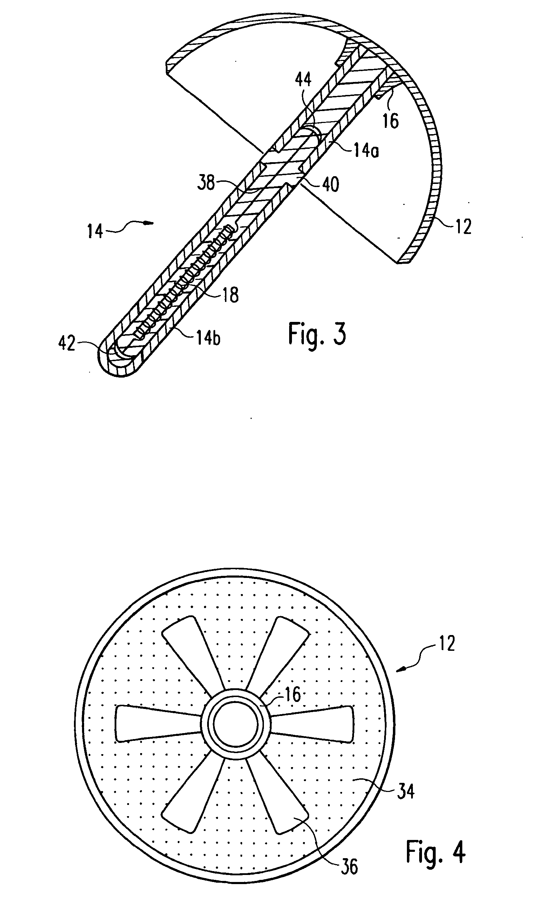

Bei der Ausführungsform gemaß Fig. 3 hat der Dom 14 zwei gegeneinander elektrisch isolierte Teile 14a, 14b. Die Teile 14a, 14b sind durch Vergußmasse 38 verbunden, die die Dornmteile 14a, 14b ausfüllt und zwischen diesen einen ringförmigen Bund 40 bildet, dessen Außenfläche mit der der Dornteile 14a, 14b fluchtet. Im einen, hier dem dem Kappenteil abgewandten Teil 14b des Doms, ist eine Aufnehmerspule 18 untergebracht. Der eine Anschluß der Aufnahmerspule 18 ist über eine Feder 42 mit dem Teil 14b gekoppelt. Der andere Anschluß ist über einen Draht und eine Feder 44 mit dem Teil 14a gekoppelt. Das Teil 14b des Doms bildet also die eine und das Teil 14a mit dem Kappenteil 12 die andere Gewebeelektrode. Wie dargestellt ist, endet das die eine Gewebeelektrode bildende, äußere Teil 14b des Doms vorzugsweise in der Nähe des Randes des vom Kappenteil 12, umschlossenen Raums oder innerhalb von diesem um eine günstige Stromverteilung in dem vom Kappenteil 12 abgedeckten Teil des Schenkelhalsknochens zu gewährleisten.

Die beiden Dornteile 14a, 14b können auch durch ein kurzes zylindrisches Zwischenstück, z. B. aus. Keramik oder PTFE verbunden werden, das zwischen den beiden Domteilen einen Bund hat, desssen Oberfläche mit der der Dornteile fluchtet.

Der eine Anschlußdraht der Aufnehmerspule geht dann durch dieses Zwischenstück. Ein solches Zwischenstück kann durch radiale Stifte in den Teilen 14a, 14b des Doms fixiert sein kann.

Die Innenfläche des Kappenteiles 12 der oben beschriebenen Ausführungsbeispiele kann, wie Fig. 4 zeigt, zum Teil mit einer punktiert dargestellten Schicht 3,4 aus Isoliermaterial abgedeckt sein. Die Isoliermaterialschicht 34 enthält oder besteht aus Hydroxylapatit (Ca₅[(PO)₄]₃OH, dem Hauptbestandteil der festen Knochensubstanz. Sie kann auch aus aufgedampftem Siliciumdioxid oder einem anderen gewebeverträglichen Isoliermaterial bestehen. Durch die isolierende Abdeckung kann die Stromdichte an den mit dem Knochen in Berührung kommenden, freigebliebenen Elektrodenbereichen 36 erhöht und/oder der Stromfluß auf bestimmte Bereiche begrenzt werden.

Wenn der Dom und/oder das Kappenteil aus elektrisch nichtleitendem Material, wie Keramik, bestehen, werden Beschichtungen aus leitendem Material vorgesehen, die den als Gewebeelektroden dienenden Teilen der oben beschriebenen Ausführungsbeispiele äquivalent sind.

## Patentansprüche

1. Hüftkopfkappenimplantat mit einem Kappenteil (12) zum Abdecken eines erkrankten Hüftgelenkkopfes, und mit einem in einen Schenkelhalsknochen einsetzbaren Dorn (14) zum Fixieren des Kappenteiles auf dem Hüftgelenkkopf, wobei Kappenteil und Dorn ein gewebeverträgliches Metall enthalten, **dadurch gekennzeichnet, dass** das Implantat eine erste und eine Zweite Gewebelektrode aufweist; dass im Dorn (14) eine Aufnehmerspule (18) mit zwei Anschlüssen, in der ein niederfrequenter Wechselstrom induzierbar ist, angeordnet ist und dass ein Anschluß der Aufnehmerspule (18) mit dem Kappenteil (12) gekoppelt ist, wobei der Kappenteil (12) zumindest einen Teil der ersten Gewebeelektrode bildet, und der andere Anschluß mit der zweiten Gewebeelektrode (22, 14, 14b) gekoppelt ist.

2. Hüftkopfkappenimplantat nach Anspruch 1, **dadurch gekennzeichnet, dass** die zweite Gewebeelektrode (22) isoliert in einer Vertiefung der Aussenseite des Dorns (14) angeordnet ist und dass die Aussenfläche der zweiten Gewebeelektode (22) mit der unvertieften Oberfläche des Dorns fluchtet (Fig. 1).

3. Hüftkopfkappenimplantat nach Anspruch 1, **dadurch gekennzeichnet, dass** der Dorn (18) und das Kappenteil (12) elektrisch gegeneinander isoliert und mit je einem Anschluss der Aufnehmerspule (18) elektrisch gekoppelt sind (Fig. 2).

4. Hüftkopfkappenimplantat nach Anspruch 1, **dadurch gekennzeichnet, dass** der Dorn zwei durch eine Isolierung gegeneinander elektrisch isolierte Teile (14a, 14b) enthält, die mit jeweils einem Anschluss der Aufnehmerspule gekoppelt sind und als Gewebeelektroden dienen (Fig. 3), wobei der eine Teil (14a) des Dorns elektrisch mit dem Kappenteil verbunden ist.

5. Hüftkopfkappenimplantat nach Anspruch 4, **dadurch gekennzeichnet, dass** die Isolierung aus Ausgußmasse (38) besteht, mit der die Teile (14a, 14b) des Dorns ausgegossen sind.

6. Hüftkopfkappenimplantat nach Anspruch 4, **dadurch gekennzeichnet, dass** die Isolierung ein isolierendes Zwischenstück enthält, das in die einander gegenüber liegenden Enden der Teile des Dorns hineinreicht und dass ein Anschluss der Aufnehmerspule hindurchgeführt ist.

7. Hüftkopfkappenimplantat nach einem der Ansprüche 4 bis 6, **dadurch gekennzeichnet, dass** die Isolierung einen ringförmigen Bund (40) aufweist, dessen Oberfläche mit der der Teile (14a, 14b) des Dorns fluchtet.

8. Hüftkopfkappenimplantat nach einem der vorangehenden Ansprüche, **dadurch gekennzeichnet, dass** ein Teil der Innenseite des Kappenteiles (12) mit einer Isolierschicht (34) versehen ist (Fig. 4).

9. Hüftkopfkappenimplantat nach Anspruch 8, **dadurch gekennzeichnet, dass** die Isolierschicht (34) Hydroxylapatit enthält.

## Claims

1. A femoral head cap implant having a cap component (12) for covering a diseased hip joint head and having a pin (14) insertable into a femoral neck bone for fixing the cap component atop the hip joint head, whereby the cap component and pin comprise a tissue-compatible metal, **characterized in that** the implant has a first and a second tissue electrode; a pick-up coil (18) having two terminals in which a low-frequency alternating current is inducable is arranged in said pin (14); one terminal of the pick-up coil (18) is coupled to said cap component (12), wherein the cap component (12) forms at least a part of the first tissue electrode; and the other terminal is coupled to the second tissue electrode (22, 14, 14a).

2. The femoral head cap implant according to claim 1, **characterized in that** the second tissue electrode (22) is disposed insulated within a recess on the exterior of said pin (14) and that the outer surface of said second tissue electrode (22) is flush with the unrecessed surface of the pin (Fig. 1).

3. The femoral head cap implant according to claim 1, **characterized in that** said pin (18) and said cap component (12) are electrically insulated from one another and each electrically coupled to one respective terminal of the pick-up coil (18) (Fig. 2).

4. The femoral head cap implant according to claim 1, **characterized in that** said pin comprises two members (14a, 14b) electrically insulated from one another by an insulation, each coupled to one respective terminal of the pick-up coil and serving as tissue electrodes (Fig. 3), one member (14a) of the pin being electrically coupled to said cap component.

5. The femoral head cap implant according to claim 4, **characterized in that** the insulation consists of sealing compound (38) with which members (14a, 14b) of said pin are filled.

6. The femoral head cap implant according to claim 4, **characterized in that** the insulation contains an insulating spacer which extends into the opposite ends of the pin members and through which passes a terminal of the pick-up coil.

7. The femoral head cap implant according to one of claims 4 through 6, **characterized in that** the insulation has an annular collar (40), the surface of which is flush with pin members (14a, 14b).

8. The femoral head cap implant according to one of the preceding claims, **characterized in that** a portion of the inner surface of the cap component (12) is provided with an insulating layer (34) (Fig. 4).

9. The femoral head cap implant according to claim 8, **characterized in that** said insulating layer (34) contains hydroxylapatite.

## Revendications

1. Implant de calotte de tête fémorale comportant un élément de calotte (12) pour couvrir une tête fémorale malade et comportant une broche (14) qui est insérable dans un os du col du fémur pour fixer l'élément de calotte sur la tête fémorale, l'élément de calotte et la broche comportant un métal qui est compatible avec du tissu, **caractérisé en ce que** l'implant comporte une première et une deuxième électrode de tissu; une bobine de captage (18) avec deux contacts, dans laquelle un courant alternatif à basse fréquence est inductible, est agencée dans la broche (14); un contact de la bobine de captage (18) est couplé à l'élément de calotte (12), l'élément de calotte (12) formant au moins une partie de la première électrode de tissu; et l'autre contact est couplé à la deuxième électrode de tissu (22, 14, 14b).

2. Implant de calotte de tête fémorale selon la revendication 1, **caractérisé en ce que** la deuxième électrode de tissu (22) est agencée de manière isolée dans un creux de la face externe de la broche (14) et la surface externe de la deuxième électrode de tissu (22) est alignée à la surface non-approfondie de la broche (Fig. 1).

3. Implant de calotte de tête fémorale selon la revendication 1, **caractérisé en ce que** la broche (18) et l'élément de calotte (12) sont isolés électriquement l'un contre l'autre et chacun couplés électriquement à un contact respectif de la bobine de captage (18), (Fig. 2).

4. Implant de calotte de tête fémorale selon la revendication 1, **caractérisé en ce que** la broche contient deux éléments (14a, 14b) isolés électriquement l'un contre l'autre par une isolation et qui sont chacun couplés à un contact respectif de la bobine de captage et qui servent comme électrodes de tissu (Fig. 3), l'un (14a) des deux éléments de la broche étant lié électriquement à l'élément de calotte.

5. Implant de calotte de tête fémorale selon la revendication 4, **caractérisé en ce que** l'isolation consiste d'une masse de scellement (38) avec laquelle les éléments (14a, 14b) de la broche sont scellés.

6. Implant de calotte de tête fémorale selon la revendication 4, **caractérisé en ce que** l'isolation contient une pièce intermédiaire isolante qui s'étend dans les extrémités opposées des éléments de la broche et au travers laquelle un contact de la bobine de captage est passé.

7. Implant de calotte de tête fémorale selon l'une des revendications 4 à 6, **caractérisé en ce que** l'isolation comporte une ceinture annulaire (40) dont la surface est alignée aux éléments (14a, 14b) de la broche.

8. Implant de calotte de tête fémorale selon l'une des revendications précédentes, **caractérisé en ce qu'**une partie de la face interne de l'élément de calotte (12) est revêtue d'une couche d'isolation (34), (Fig. 4).

9. Implant de calotte de tête fémorale selon la revendication 8, **caractérisé en ce que** la couche d'isolation (34) contient de l'hydroxyapatite.
